# EUROPÄISCHE PATENTSCHRIFT

(11) **EP 0 587 118 B1**
(45) Veröffentlichungstag und Bekanntmachung des Hinweises auf die Patenterteilung: **13.01.1999**
(21) Anmeldenummer: 93114340.8
(22) Anmeldetag: 07.09.1993
(51) Int. Cl.: A61F 13/54, A61L 15/48, D04H 11/08

(54) **Flüssigkeitsdurchlässige Decklage für eine körperflüssigkeitsabsorbierende Ware**
Liquid pervious topsheet for a body fluid absorbent article
Couche supérieure perméable aux liquides pour un article absorbant des fluides du corps

(30) Priorität: 07.09.1992 JP 238417/92
(43) Veröffentlichungstag der Anmeldung: 16.03.1994
(73) Patentinhaber: Mitsui Chemicals, Inc., Tokyo 100 (JP); UNI-CHARM CORPORATION, Kawanoe-shi Ehime-ken (JP)
(72) Erfinder: Takai, Hisashi, Kawanoe-shi, Ehime-ken (JP); Kido, Tsutomu, Kawanoe-shi, Ehime-ken (JP)
(74) Vertreter: Sperling, Rüdiger, Dipl.-Ing.

(56) Entgegenhaltungen:
- EP-A- 0 409 535
- AT-B- 351 156
- DE-A- 3 517 640
- GB-A- 2 180 271

## Beschreibung

### Hintergrund der Erfindung

Die vorliegende Erfindung bezieht sich auf eine in einer flüssigkeitsabsorbierenden Einwegware wie Damenbinden und Einwegwindeln verwendete flüssigkeitsdurchlässige Decklage.

In einer körperflüssigkeitsabsorbierenden Einwegwaren wird konventionellerweise oft eine perforierte Plastiklage oder ein Vliesstoff als Material für eine flüssigkeitsdurchlässige Decklage verwendet. Im Fall der perforierten Plastiklage ist die Lage mit Flüssigkeitsdurchlässen versehen, die sich von der Oberseite zur Unterseite durch die Lage hindurch erstrecken, und die unteren Öffnungen der entsprechenden Flüssigkeitsdurchlässe sind kontaktierend mit einem absorbierenden Kern angeordnet, so daß die ausgeschiedenen Körperflüssigkeiten schnell in den absorbierenden Kern geleitet werden können. Eine derartige Technik ist beispielsweise aus der JPB Nr. 57-17081 (AT-351156), die konisch verlaufende Flüssigkeitsdurchlässe offenbart, und aus der gattungsgemäßen JPA 60-259261 (DE OS 35 17 640), die zylindrische Flüssigkeitsdurchlässe offenbart, bekannt. Der Gebrauch von Vliesstoff wird andererseits dem Gebrauch der Plastiklage bevorzugt, da der Vliesstoff ein weiches Tragegefühl und daher ein komfortables Tragen vermittelt.

Während die konventionelle Technik ihre oben erwähnten Vorteile auf ihre eigene Weise bereitstellt, hat der Gebrauch der perforierten Plastiklage Nachteile, die oft von dem Konsumenten abgelehnt werden, insbesondere das der Plastiklage eigene schlüpfrige Tragegefühl und die glänzende Erscheinung, so daß ein akuter Bedarf für eine verbesserte Decklage besteht, die sowohl in dem Gefühl als auch in der Erscheinung einem Stoff so ähnlich wie möglich ist. Im Fall der Verwendung von Vliesstoff als Material der Decklage können die ausgeschiedenen Körperflüssigkeiten so schnell wie bei der Verwendung der Plastiklage in den absorbierenden Kern geleitet werden, allerdings hat der Vliesstoff nur eine geringe Fähigkeit, die obere Oberfläche der Lage trocken zu halten, nachdem die Körperflüssigkeiten in den absorbierenden Kern geleitet worden sind, d.h. es mangelt an einem trockenen Gefühl. In dieser Hinsicht wird eine echte Verbesserung verlangt. Um den besten Gebrauch der von diesen konventionellen Materialien bereitgestellten Vorteile zu machen, und deren Nachteile zu kompensieren, wurde bereit beispielsweise in JPA 3-51355 vorgeschlagen, eine Lage eines blasgeschmolzenen Vliesstoffs zu verwenden, der gebildet wird, indem die thermoplatischen Fasern einem Schweißprozeß unterzogen werden. Obwohl es die in dieser Schrift offengelegte Technik ermöglicht, eine Decklage zu bilden, die ein stoffähnliches, weiches Gefühl und eine weniger glänzende Erscheinung vermittelt und die mit Flüssigkeitsdurchlässen versehen ist, ist der blasgeschmolzene Vliesstoff im allgemeinen durch seine relativ geringe Feinheit und der damit verbundenen geringen Kompressionseleastizität gekennzeichnet, so daß die Flüssigkeitsdurchlässe schnell unter dem Körpergewicht des Benutzers kollabieren, wobei die unteren Öffnungen der Flüssigkeitsdurchlässe verdreht werden, so daß die Körperflüssigkeiten an einer schnellen Ableitung in den absorbierenden Kern gehindert werden.

Es ist daher eine Aufgabe der Erfindung die erwähnten Probleme zu lösen, indem eine Decklage aus einer aus blasgeschmolzenem Vliesstoff zusammengesetzten oberen Deckschicht gebildet wird, die mit Flüssigkeitsdurchlässen versehen ist, deren Kollabieren oder Verdrehen vermieden wird.

### Zusammenfassung der Erfindung

Die oben gestellte Aufgabe wird gemäß der Erfindung durch die Merkmale des Hauptanspruchs gelöst.

Die Decklage gemäß der Erfindung ermöglicht es, daß die Körperflüssigkeiten schnell in den absorbierenden Kern geleitet werden und bietet ein stoffähnliches Tragegefühl und Erscheinung, da die Decklage eine aus blasgeschmolzenem Vliesstoff hergestellte, mit Durchlässen versehene obere Deckschicht und eine untere fibröse Schicht aufweist, die mit der oberen Deckschicht verbunden ist, um eine Konfiguration der Flüssigkeitsdurchlässe zu stabilisieren, so daß diese Flüssigkeitsdurchlässe nicht leicht kollabieren und die unteren Öffnungen der Flüssigkeitsdurchlässe nicht wesentlich verdreht werden.

### Kurze Beschreibung der Zeichnungen

Die Erfindung wird beispielhaft unter Bezugnahme auf die beigefügten Zeichnungen erklärt, in denen
Fig.1 eine perspektivische Ansicht mit einem Teilschnitt einer Damenbinde unter Verwendung einer Decklage der Erfindung ist; und
Fig. 2 einen Querschnitt längs der Linie X-X der Fig. 1 ist, der einen Ausschnitt der Damenbinde in einem vergrößerten Maßstab zeigt.

### Beschreibung der bevorzugten Ausführungsform

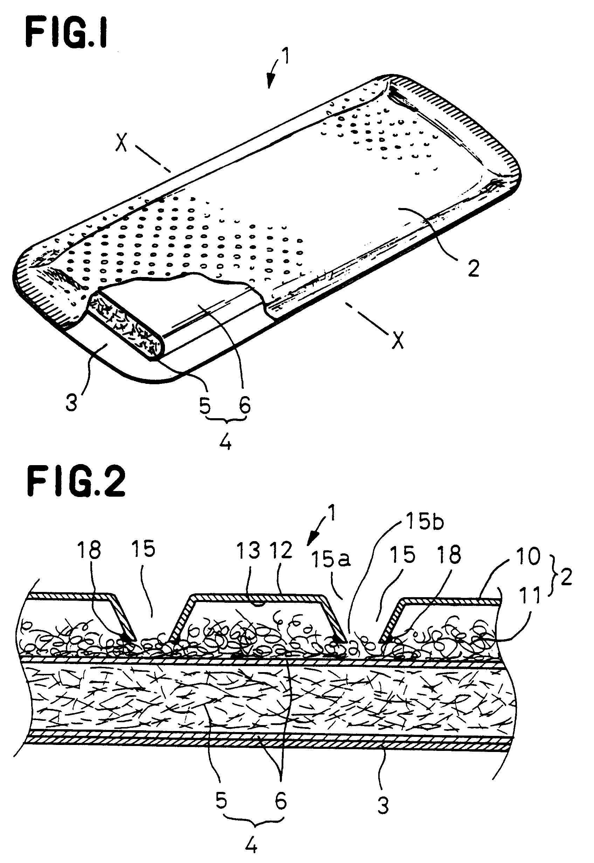

Fig. 1 zeigt in perspektivischer Ansicht mit einem Teilschnitt eine Damenbinde 1, die eine Decklage 2 gemäß der Erfindung verwendet. Wie dargestellt umfaßt die Damenbinde 1 die flüssigkeitsdurchlässige Decklage 2, eine flüssigkeitsundurchlässige rückseitige Lage 3 und einen absorbierenden Kern 4, der zwischen diesen Lagen 2, 3 eingefaßt ist, wobei Teile der Lagen 2, 3, die sich über die Randkanten des absorbierenden Kerns 4 hinaus erstrecken, fest miteinander verbunden sind.

Fig. 2 zeigt die Decklage 2 in größerem Detail an Hand eines Querschnitts entlang der Linie X-X der Fig. 1 in einem größeren Maßstab. Die Decklage 2 ist aus einer oberen Deckschicht 10, die zusammengeschweißte thermoplastische Fasern aufweist, und einer unteren fibrösen Schicht 11 zusammengesetzt, die der oberen Deckschicht 10 unterliegt und dazu dient, die obere Deckschicht 10 mit einer Steifigkeit zu versehen. Die obere Deckschicht 10 ist mit Flüssigkeitsdurchlässen 15 versehen, wobei sich jeder von der Oberseite 12 zur Unterseite 13 hindurch erstreckt und obere und untere Öffnungen 15a, 15b definiert. Diese Durchlässe 15 machen die Deckschicht 10 flüssigkeitsdurchlässig. Die darunterliegende fibröse Schicht 11 bedeckt die entsprechenden Öffnungen 15b bis zu einem Maße, daß die Flüssigkeitsdurchlässigkeit der Deckschicht 10 erhalten bleibt, und ist mit ihnen mit einem heißschmelzenden Kleber zumindest angrenzend an die unteren Öffnungen 15b mit einer Teilzahl der Flüssigkeitsdurchlässe 15 verbunden. Die untere fibröse Schicht 11 dient dazu, die obere Schicht 10 mit einer Kompressionselastizität zu versorgen und dadurch die Flüssigkeitsdurchlässe 15 von der Unterseite zu verstärken, so daß die Flüssigkeitsdurchlässe 15 gegen ihr Kollabieren wirkungsvoll geschützt werden können, und daß die unteren Öffnungen 15b dieser Durchlässe 15 vor ihrer Verdrehung geschützt werden können. Zusätzlich steht die untere fibröse Schicht 11 in Kontakt mit dem absorbierenden Kern 4 und unterstützt die von den Flüssigkeitsdurchlässen 15 geleiteten Körperflüssigkeiten dabei in den absorbierenden Kern 4 durch eine Kapillarwirkung geleitet zu werden. Die einzelnen Fasern der unteren fibrösen Schicht 11 gelangen oft teilweise in die Flüssigkeitsdurchlässe 15 und diese Fasern erzeugen ebenfalls die kapillare Wirkung, die dazu dient die Körperflüssigkeiten in den absorbiernden Kern 4 zu ziehen.

Die obere Deckschicht 10 kann aus blasgeschmolzenem Vliesstoff hergestellt werden, der ein Gewicht pro Einheitsfläche von 10 bis 100 g/m2 hat, und der beispielsweise aus thermopolastischen Fasern aus Olefin, Polyester oder Polyamid besteht. Im allgemeinen enthält der blasgeschmolzene Vliesstoff Fasern relativ geringer Deniers und macht es leicht möglich, sowohl ein angenehmes Tragegefühl als auch ein wenig glänzendes Erscheinen zu vermitteln. Der Vliesstoff diesen Typs kann entweder luftdurchlässig oder luftundurchlässig hergestellt werden, was von verschiedenen Faktoren abhängt, beispielsweise dem Gewicht pro Einheitsfläche und ob der Vliesstoff einer zweiten Wärmebehandlung unter einem Druck unterworfen wird oder nicht. Der blasgeschmolzenen Vliesstoff kann als Material für die Decklage 10 verwendet werden, ob er nun luftdurchlässig oder luftundurchlässig ist. Um den blasgeschmolzenen Vliesstoff mit den Flüssigkeitsdurchlässen 15 zu versehen, werden nach dem in der JPA 3-51355 offenbarten Verfahren blasgeschmolzene Fasern gegen eine Spritzformplatte, die Öffnungen einer vorbestimmten Querschnittsform aufweist, in einem Herstellungsverfahren des Vliesstoffs geblasen, wobei die Flüssigkeitsdurchlässe 15 gebildet werden. Die unteren Enden der entsprechenden Flüssigkeitsdurchlässe 15 weisen Unebenheiten auf, d.h. Kerben (Unregelmäßigkeiten), die bleiben können wie sie sind oder, falls notwendig, sauber entfernt werden.

Die untere fibröse Schicht 11 kann durch eine bekannte thermoplastische Faser gebildet sein, beispielsweise durch Polyethylen- oder Polyproylenfasern mit einer Feinheit von 1 bis 10 Deniers, einem Gewicht pro Einheitsfläche von 10 bis 40 g/m2 und einer Dichte von 0,01 bis 0,1 g/cm3. Damit die untere fibröse Schicht 11 ihre Anfangselastizität hinsichtlich der Kompression selbst nach der Befeuchtung mit den Körperflüssigkeiten beibehalten kann, kann die untere fibröse Schicht 11 mit einer Menge eines sogenannten schweißabsorbierenden Kunstharzes gemischt werden, das aus hydrophilen Fasern wie Rayonfasern oder Polyesterfasern zusammengesetzt ist, deren Oberfläche hydrophil gemacht wurde, so daß die Ableitung der Körperflüssigkeiten in den absorbierenden Kern 4 erleichtert wird. Falls es notwendig ist, wird die untere fibröse Schicht 11 mit einem entsprechenden Hydrophilie erzeugen-dem Mittel behandelt (grenzflächenaktiver Stoff).

Das Material für die untere fibröse Schicht 11 kann weiterhin aus einer Gruppe ausgewählt werden, die schmelzgeblasenen Vliesstoff, schmelzgebundenen Vliesstoff, bindemittelgebundenen Vliesstoff, Vliesstoff mit wasserstrahlverfilzten Fasern und einfach akkumulierte Fasern aufweist, die in der Lage sind eine gewünschte plattenartige Form beizubehalten. Um die Flüssigkeitsdurchlässe 15 mit einer größeren Stabilität zu unterstützen, sollten bevorzugterweise Hohlräume zwischen den Fasern vorhanden sein, die es zumindestens teilweise erlauben, daß die unteren Enden der entsprechenden Flüssigkeitsdurchlässe 15 in den oberen Abschnitt der unteren fibrösen Schicht 11 eindringen.

Um die obere Deckschicht 10 und die untere fibröse Schicht 11 miteinander zu verbinden, werden diese Schichten 10, 11 aneinander gepreßt, wobei ein heißer, geschmolzener Kleber oder ähnliches auf der oberen Deckschicht 10 um die entsprechenden Öffnungen 15b herum und auf der unteren fibrösen Schicht 11 aufgebracht ist. Alternativ können die entsprechenden Materialfasern, die die oberen Deckschicht 10 und die untere fibröse Schicht 11 bilden, miteinander verschweißt werden, um einen ähnlichen Verbindungseffekt zu erreichen.

Bevorzugt sind die Flüssigkeitsdurchlässe 15 für einen Durchmesser von 0,2 bis 3mm, insbesondere von 0,7 bis 2mm an der oberen Öffnung 15a, ein Flächenverhältnis der Kehle von 10% bis 70%, eine Höhe von 0,3 bis 5mm und einem Durchmesser der unteren Öffnung 15b ausgelegt, der 30% oder mehr des Durchmessers der oberen Öffnung 15a beträgt. Eine derartige Dimensionierung dient nicht nur einer reibungslosen und schnellen Leitung eines Stroms von Körperflüssigkeiten in den absorbierenden Kern 4, sondern auch zur Vermeidung eines merklichen Rückstroms.

Die flüssigkeitsundurchlässige, rückseitige Schicht 3 kann aus einer Polyethylenschicht gebildet werden, und der absorbierende Kern 4 kann aus einem Gemisch aus Flusenstoff und superabsorbierendem Polymer bestehen, der mit Seidenpapier bedeckt ist.

Die erfindungsgemäße Decklage ermöglicht das schnelle Ableiten der Körperflüssigkeiten in den absorbiernden Kern und ermöglicht ein stoffähnliches Tragegefühl und Erscheinung, da die Decklage eine obere Deckschicht aus mit Flüssigkeitsdurchlässen versehenen blasgeschmolzenem Vliesstoff und eine untere fibröse Schicht aufweist, die mit der oberen Deckschicht verbunden ist, um eine Konfiguration der Flüssigkeitsdurchlässe zu stabilisieren, so daß diese Flüssigkeitsdurchlässe nicht einfach kollabieren und daß die unteren Öffnungen dieser Flüssigkeitsdurchlässe nicht wesentlich verdreht werden.

## Patentansprüche

1. Decklage einer Körperflüssigkeit-absorbierenden Ware mit:
a) einer oberen Deckschicht (10) aus einem Schmelz-Blas-Vliesstoff mit einem Gewicht von 10 bis 100 g/m² pro Einheitsfläche und einer Vielzahl von sich nach unten durch die Deckschicht hindurcherstreckenden Flüssigkkeitsdurchlässen (15), von denen jeder ein oberes Ende mit einer Öffnung(15a) mit einem Durchmesser von 0,2 bis 3 mm und ein unteres Ende mit einer Öffnung(15b) mit einem geringeren Durchmesser aufweist;
b) einer unter der oberen Deckschicht angeordneten unteren fibrösen Schicht (11), mit einer Feinheit von 1 bis 10 Denier, einem Gewicht von 10 bis 40 g/m² und einer Dichte von 0,01 bis 0,1 g/cm³, wobei zumindest die unteren Öffnungen(15b) der sich nach unten erstreckenden Flüssigkeitsdurchlässe mit der unteren Schicht (11) verbunden sind, so daß die sich nach unten erstreckenden Flüssigkeitsdurchlässe(15) einem Kollabieren und die unteren Öffnungen(15b) einer Verdrehung widerstehen können.

2. Decklage nach Anspruch 1, wobei die Flüssigkeitsdurchlässe(15) eine Höhe von 0,3 bis 5 mm aufweisen.

3. Decklage nach Anspruch 1 oder 2, wobei das die untere Schicht (11) bildende Faservlies nur aus hydrophoben synthetischen Fasern besteht.

4. Decklage nach einem der Ansprüche 1 bis 3, wobei die untere fibröse Schicht (11) lediglich aus hydrophilen synthetischen Fasern besteht.

5. Decklage nach mindestens einem der Ansprüche 1 bis 3, wobei die untere fibröse Schicht (11) (a) hydrophile oder (b) synthetische Fasern aufweist, und wobei deren Oberfläche hydrophil gemacht wurden oder (c) eine Mischung aus (a) und (b) ist.

6. Decklage nach einem der Ansprüche 1 bis 5, wobei die untere fibröse Lage mit einer Agens behandelt wurde, das Hydrophilität erzeugt.

## Claims

1. Topsheet of body-fluid absorptive goods having:
(a) an upper cover layer (10) made of melt blow nonwoven fabric having a weight per unit area of 10 to 100 g/m² and a number of liquid passages (15) extending downwards through the cover layer, each of said passages having an upper end with an opening (15a) with a diameter of 0.2 to 3 mm and a lower end with an opening (15b) with a smaller diameter;
(b) a lower fibrous layer (11) underlying said upper cover layer and having a fineness of 1 to 10 deniers, a weight of 10 to 40 g/m², and a density of 0.01 to 0.1 g/cm³, wherein at least said lower openings (15b) of the downward extending liquid passages are bonded to said lower layer (11) so that the downward extending liquid passages (15) are able to resist collapsing and the lower openings (15b) are able to resist twisting.

2. Topsheet as claimed in claim 1, wherein said liquid passages (15) have a height of 0.3 to 5 mm.

3. Topsheet as claimed in claim 1 or 2, wherein said fibrous nonwoven fabric forming said lower layer (11) solely consists of hydrophobic synthetic fibers.

4. Topsheet as claimed in one of claims 1 to 3, wherein said lower fibrous layer (11) consists only of hydrophilic synthetic fibers.

5. Topsheet as claimed in at least one of claims 1 to 3, wherein said lower fibrous layer (11) comprises (a) hydrophilic or (b) synthetic fibers, and wherein their surface has been made hydrophilic, or comprises (c) a mixture of (a) and (b).

6. Topsheet as claimed in one of claims 1 to 5, wherein said lower fibrous layer has been treated with a hydrophilicity giving agent.

## Revendications

1. Couche de revêtement pour article absorbant les fluides corporels, comprenant :
a) une couche de couverture supérieure (10) en non tissé venu de fusion-soufflage, d'un poids de 10 à 100 g/m² par unité de surface, et une multiplicité de passages à liquides (15) cheminant vers le bas au travers de la couche de couverture, dont chacun présente une extrémité supérieure comportant une ouverture (15a) d'un diamètre de 0,2 à 3 mm et une extrémité inférieure comportant une ouverture (15b) de plus petit diamètre;
b) une couche inférieure fibreuse (11) sous-jacente à la couche de couverture supérieure, et d'une finesse de 1 à 10 deniers, d'un poids de 10 à 40 g/m² et d'une densité de 0,01 à 0,1 g/cm³, les ouvertures inférieures (15b), au moins, des passages à liquides qui s'étendent vers le bas étant raccordées à la couche inférieure (11), en sorte que les passages à liquides (15) qui s'étendent vers le bas puissent résister à un affaissement, et les ouvertures inférieures (15b), à une torsion.

2. Couche de revêtement suivant la revendication 1, dans laquelle les passages à liquides (15) présentent une hauteur de 0,3 à 5 mm.

3. Couche de revêtement suivant l'une des revendications 1 ou 2, dans laquelle le non tissé formant la couche inférieure (11) se compose uniquement de fibres synthétiques hydrophobes.

4. Couche de revêtement suivant l'une des revendications 1 à 3, dans laquelle la couche inférieure fibreuse (11) se compose uniquement de fibres synthétiques hydrophiles.

5. Couche de revêtement suivant au moins l'une des revendications 1 à 3, dans laquelle la couche inférieure fibreuse (11) comporte des fibres (a) hydrophiles, ou (b) synthétiques et dont la surface a été rendue hydrophile, ou, (c) est un mélange de (a) et (b).

6. Couche de revêtement suivant l'une des revendications 1 à 5, dans laquelle la couche inférieure fibreuse a été traitée avec un agent qui crée une hydrophilie.
